**Europäisches Patentamt**

**European Patent Office**

**Office européen des brevets**

(11) Veröffentlichungsnummer: **0 252 407**

**A2**

(12) **EUROPÄISCHE PATENTANMELDUNG**

(21) Anmeldenummer: **87109363.9**

(22) Anmeldetag: **30.06.87**

(51) Int. Cl.⁴: **B01J 2/10**

(30) Priorität: **11.07.86 DE 3623321**

(43) Veröffentlichungstag der Anmeldung:
**13.01.88 Patentblatt 88/02**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Bücheler, Manfred, Dipl.-Ing.**
**Lorkenhöhe 49**
**D-5063 Overath(DE)**
Erfinder: **Schmoll, Josef, Dipl.-Ing.**
**Irlenweg 6**
**D-5632 Wermelskirchen 2(DE)**
Erfinder: **Metzger, Karl-Ludwig, Dr.**
**Roggendorfstrasse 49**
**D-5000 Köln 80(DE)**
Erfinder: **Luchtenberg, Helmut, Dr.**
**Windmühlenstrasse 11a**
**D-5216 Niederkassel(DE)**

(54) **Verfahren zur kontinuierlichen Herstellung von sphärischen Granulaten.**

(57) Sphärische Granulate werden kontinuierlich hergestellt, indem man in einem Rotor-Granulator das in Form von Pulver und Keimkorn vorliegende Ausgangsmaterial durch Besprühen mit einer Flüssigkeit granuliert und das fertige Granulat dem Granulator kontinuierlich entnimmt. Neben dem Pulver und der Granulierflüssigkeit wird dabei auch das Keimkorn kontinuierlich zudosiert. Besonders bewährt hat sich eine Ausführungsform, bei der mittels einer Entnahmevorrichtung (10) das Granulat an der Peripherie einer am Innenumfang des Granulators (1) angehäuften Schüttguttrombe (9) abgezogen wird. Dabei werden die Betriebsbedingungen des Granulators so eingestellt, daß den Granulat-Partikeln in der Trombe (9) eine zirkulierende Bewegung aufgezwungen wird. Auf diese Weise ist eine klassierende Entnahme des Granulats möglich. Das fertige Granulat weist also bereits im großen und ganzen eine einheitliche Korngröße auf. Die Klassierung kann weiter verbessert werden, wenn dem Rotor-Granulator extern ein Apparat (15) nachgeschaltet wird, in dem eine Fraktionierung nach Korngrößen erfolgt.

FIG. 1

## Verfahren zur kontinuierlichen Herstellung von sphärischen Granulaten

Die Erfindung betrifft ein Verfahren zur kontinuierlichen Herstellung von sphärischen Granulaten mit Korngrößen zwischen 0,1 und 2 mm, ausgehend von Pulvern unter Zuhilfenahme einer Granulierflüssigkeit.

Es ist bekannt, Granulate durch Abrollen der Partikeln auf einer schiefen Ebene, z.B. Teller oder Trommel, unter Ausnutzung der Schwerkraft zu erzeugen (Chem. Ing. Techn. 50 (1972), Nr. 7, 518-524). Diese Verfahren beschränken sich jedoch auf mittelere Korngrößen oberhalb von ca. 2 mm.

Ein weiteres Aufbaugranulierverfahren stellt die sogenannte Rotorgranulation dar.

Dieses bekannte Verfahren der Rotorgranulation stellt sich wie folgt dar:

a) Einheit nach Stand der Technik (diskontinuierlich)

Die Vorrichtung besteht aus einem zylindrischen Behälter mit vertikaler Zentralachse, dessen Boden um die Zentralachse rotiert (Drehzahlbereich von 50 - 500 min$^{-1}$). Der Zylindermantel ist feststehend. Das Verhältnis Durchmesser zu Höhe beträgt etwa 2,5:1, bei einem Durchmesser zwischen 300 -1500 mm (übliche Größen).

Für die Zuführung des zu granulierenden Produktes im folgenden Pulver genannt, ist ein Feststoffdosierorgan (z.B. Schneckendosierer) oberhalb des Zylinders angeordnet. Mit Hilfe einer Dosierpumpe (z.B. Zahnradpumpe) wird die Granulierflüssigkeit über eine Dispergiereinheit (meistens eine Zweistoffdüse) auf (oder in) den Granulatorinhalt gesprüht.

Zur Vermeidung von Produktverlust und gegebenenfalls für die Trocknung der Granulate kann Luft in den Ringspalt zwischen dem rotierenden Granulatorboden und dem Zylindermantel eingeblasen werden.

b) Verfahrensablauf nach Stand der Technik (diskontinuierlich)

Zu Beginn des Granulationsvorganges wird ein geeig netes Produkt (im folgenden Startkorn genannt), mit einer Korngröße zwischen 0,2 -0,4 mm, in den Granulator eingefüllt. Als Startkorn eignet sich entweder ein Hilfsstoff, welcher im verlangten Korngrößenbereich vorliegt (z.B. Milchzucker grob für pharmazeutische Produkte), oder ein in einem vorgeschalteten Granulationsprozeß (z.B. Mischgranulation) erzeugtes Granulat des zu granulierenden Pulvers.

Dem Granulatorinhalt (zu Beginn das Startkorn) wird durch die Drehbewegung des Granulatorbodens eine Radialbewegung in Richtung des feststehenden Zylindermantels (Granulatorgehäuses) verliehen. Durch nachfolgendes Granulat wird der Granulatorinhalt am Zylindermantel nach oben gedrückt und häuft sich dort trombenförmig an. Da der beschriebenen, im wesentlichen radialen Bewegung der Partikel eine Geschwindigkeitskomponente im Umfangsrichtung überlagert ist, bewegen sich die einzelnen Granulate in einer spiralähnlichen Bahn entlang des Zylindermantels. Bedingung für diesen Verfahrensablauf ist, daß die Rotordrehzahl in einem Bereich eingestellt wird, der ein Abrollen der Einzelpartikel in der eben beschriebenen Form ermöglicht, und somit jeder Partikel die gleiche Möglichkeit zur Anlagerung von Pulver gegeben wird.

Das Pulver, welches auf das Keimkorn aufgranuliert werden soll, wird gleichzeitig mit der Granulierflüssigkeit in den Granulator dosiert. Hierbei ist das Verhältnis von Feststoff zu Flüssigkeit für die Aufrechterhaltung der erforderlichen Granulatbewegung von entscheidender Bedeutung und kann u.U. vom Füllstand im Granulator abhängig sein.

Ist der maximale Füllstand im Granulator erreicht (im allgemeinen etwa die vierfache Masse des Anfangsinhaltes) wird das Granulat entnommen.

(Pharm. Ind. 48, In. 2 (1086), 187-192; Chem.-Ing. Tech. 51 (1979, In. 4, S. 277-182; Pharm.Ind. 45, In. 7 (1983), 722-728).

Bei diesem diskontinuierlich arbeitenden Verfahren kann aus physikalischen Gründen in einem Aufbaugranulierschritt der Durchmesser des bei diesem Verfahren vorzulegenden Starterkorns maximal verdoppelt werden. Ein großer Nachteil dieses Verfahrens besteht darin, ein geeignetes Starterkorn mit dem notwendigen Korndurchmesser und der engen Verteilungsbreite zu realisieren. Für die Herstellung des Starterkorns ist entweder ein separater Granulierschritt erforderlich, oder es muß ein geeigneter Hilfsstoff mit dem erforderlichen Kornspektrum gefunden werden.

Zur Verwirklichung eines engen Kornspektrums sind weiterhin Fraktionierschritte erforderlich, und zum weiteren Aufbau der Granulate muß der Erstansatz portionsweise weiter aufgranuliert und wieder fraktioniert werden, so daß viele nicht oder - schlecht automatisierbare Einzelschritte erforderlich sind, um ein Granulat ausreichender Größe mit akzeptabler Ausbeute zu erlangen.

Durch die diskontinuierliche Aufbaugranulation mit sich zeitlich änderndem Granulierverhalten, z. B. durch Füllstanstandsänderung im Granulator, ist ein hoher Beobachtungs-und Nachregelbedarf erforderlich. Das chargenweise anfallende Endprodukt erfordert eine ebenfalls chargenweise Nachbehandlung, z.B. Trockung. Ein weiterer großer Nachteil ist der durch den hohen Anteil an Nebenzeiten stark verminderter spezifischer Durchsatz des Granulators.

Der Erfindung liegt nun die Aufgabe zugrunde, das Verfahren zur Herstellung von sphärischen Granulaten unter wirtschaftlichen Aspekten zu verbessern, wobei gleichzeitig eine hohe Produktqualität (Homogenität und enge Korngrößenverteilung der Granulate) gewährleistet sein soll. Insbesondere sollen die bisher üblichen, zeitaufwendigen Einzeloperationen entfallen und gegebenenfalls durch einen selbstregelnden Verfahrensablauf ersetzt werden.

Diese Aufgabe wird erfindungsgemäß dadurch gelöst, daß neben dem Pulver und der Granulierflüssigkeit auch das Keimkorn dem Rotorgranulator kontinuierlich zudosiert wird und das Granulat dem Granulator kontinuierlich entnommen wird.

Dabei werden die Betriebsbedingungen (stationäre Beschickung und Rotordrehzahl) vorteilhaft so eingestellt, daß sich im Gleichgewichtszustand am Innenumfang des Granulators eine Schüttguttrombe ausbildet, und die darin befindlichen Partikel sich entlang des Zylindermantels auf einer spiralähnlichen Bahn bewegen. Überraschend hat sich nun herausgestellt, daß man bereits im Granulator eine Klassierung des Granulats nach der Korngröße erreichen kann, wenn man das Granulat im stationären Betriebszustand an der Peripherie der am Innenumfang des Granulators angehäuften Schüttguttrombe mittels einer Entnahmevorrichtung, z.B. mit einem Schälrohr oder Überlauf, kontinuierlich abzieht. Der Vorteil dieser Verfahrensweise liegt also darin, daß das Granulat bereits nach Korngröße getrennt dem Granulator entnommen wird. Stellt man noch höhere Ansprüche an die hierbei schon sehr gute Verteilungsbreite des Kornspektrums, z. B. auf 1,0 - 1,2 mm, so wird in einem weiteren externen Verfahrensschritt entweder diese schon vorklassierte Granulatfraktion oder ein beliebiger Teilstrom kontinuierlich einer Fraktioniereinheit zugeführt.

Vorzugsweise wird die so gewonnene Grobfraktion dann zerkleinert und dem Granulator kontinuierlich als Keimfraktion zugeführt. Während bei der offenen Betriebsweise, d.h. bei externer Keimzugabe, Kornspektrum und Verteilungsbreite durch Keimkornmengenstrom und Verweilzeit variiert werden können, stellt sich bei dieser geschlossenen Betriebsweise, d.h. mit Rückführung, das durch periphere Fraktionierung vorgegebene Kornspektrum selbstregulierend ein.

Die erhaltene Gutfraktion kann gegebenenfalls online kontinuierlich getrocknet (z. B. Wirbelschicht; Mikrowellen-, Hochfrequenz-Trocknung usw.) werden.

Die Korngröße des fertigen Granulates kann durch die Keimdosierrate bzw. durch die Partikelanzahl des Keimkornes gesteuert werden.

Durch die Festlegung der Trennkorngröße bei der externen Fraktionierung und Rückführung der Keimfraktion wird erreicht, daß sich das entstehende Kornspektrum automatisch einstellt.

Durch die externe Zerkleinerung von Überkorn und komplette Rückführung kann man eine Ausbeute an Granulat der vorgegebenen Kornverteilung von praktisch 100 % erzielen.

Wenn es das zu granulierende Stoffsystem erforderlich macht, kann das rückgeführte Keimkorn ebenfalls online getrocknet werden. Ebenfalls kann während des Granuliervorganges im Rotorgranulator eine Vortrocknung zum Zwecke des Auskristallisierens von Festkörperbrücken erforderlich oder von Vorteil sein.

Eine spezielle Ausführungsform des erfindungsgemäßen Ver. fahrens stellt sich wie folgt dar:

Für die Inbetriebnahme des kontinuierlichen Prozesses ist es zunächst erforderlich, in einem Vorlauf Granulat der gewünschten Korngrößenverteilung zu erstellen (z. B. mit dem auf S. 2 beschriebenen Verfahren). Die zum Erreichen des stationären Zustandes im Granulator erforderliche Zeit (und gegebenenfalls das damit verbundene Auschußprodukt) ist entscheidend von den Eigenschaften des vorgelegten Granulates abhängig (z. B. Korngrößenverteilung, Gutsfeuchte, usw.).

Ausgehend vom Startinhalt des Granulators werden gleichzeitig und kontinuierlich Pulver, Keimkorn und Granulierflüssigkeit in den laufenden Granulator dosiert. Durch gleichzeitige Entnahme von Granulaten bleibt der Füllstand im Granulator konstant.

Die Entnahme, welche entweder durch Abwurf über den Rand oder durch eine Entnahmesonde (Schälrohr) erfolgt, wirkt, wie oben beschrieben, klassierend, wenn die Rotordrehzahl in bestimmten Grenzen liegt. Diese Grenzen hängen jeweils vom dem zu granulierenden Produkt ab und können im Einzelflal leicht experimentell ermittelt werden.

Für diese spezielle Ausführungsform ergeben sich z.B. folgende Möglichkeiten:

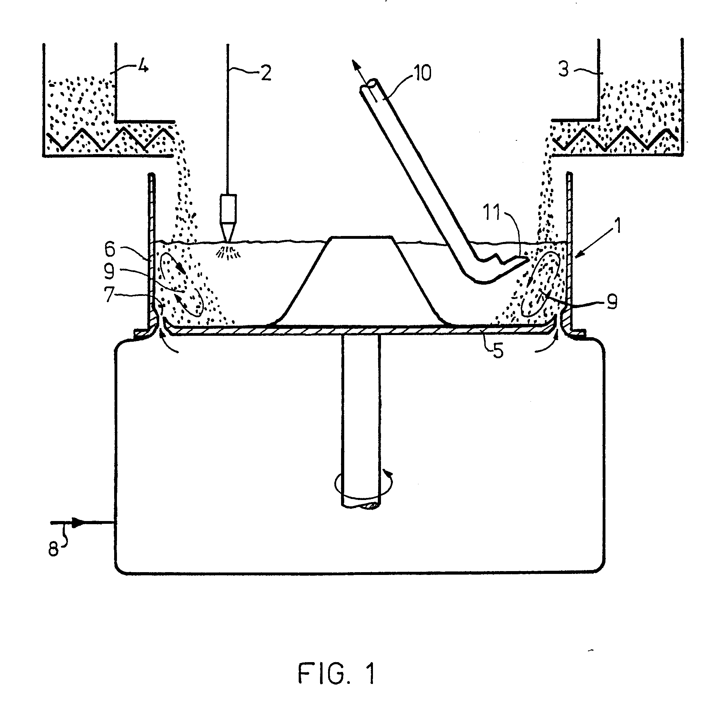

## a) Verfahrensablauf bei externer Keimkornzugabe - (Fig. 1)

Pulver und Keimkorn werden in einem vorgegebenen Massenverhältnis bei gleichzeitiger Zugabe von Granulierflüssigkeit in den Granulator 1 dosiert. Die Granulierflüssigkeit (Bindemittel) wird durch eine Düse 2 auf das Granulat aufgesprüht. Zur Eindosierung des zu granulierenden Pulvers und des Keimkornes werden handelsübliche Schneckendosierer 3 und 4 verwendet. Durch den zwischen Rotor 5 und Gehäuse 6 verbleibenden Spalt 7 wird eine verhältnismäßig geringe Menge Luft (8) eingeblasen. Dadurch wird verhindert, daß durch den Ringspalt 7 Granulatmaterial in den darunter befindlichen Raum austritt. Durch entsprechende Einstellung der Rotordrehzahl läßt sich leicht erreichen, daß sich im stationären Betriebszustand an der Innenwand des Gehäuses 6 eine Schüttguttrombe 9 ausbildet. In dieser Trombe bewegen sich die Granulatpartikel, wie oben beschrieben, spiralförmig entlang der Innenoberfläche des Granulators 1. Im Querschnitt entspricht dies einer zirkulierenden Bewegung der Teilchen (durch Pfeile innerhalb der Schüttguttrombe 9 angedeutet). Dabei hat sich überraschenderweise herausgestellt, daß an der Peripherie der Schüttguttrombe 9 im Gleichgewichtszustand überwiegend die Granulatkörner mit maximalem Durchmesser zirkulieren, während im Inneren der Trombe eine breite Korngrößenverteilung vorherrscht. Dieser Befund kann vorteilhaft für eine klassierende kontinuierliche Entnahme des fertigen Granulats ausgenutzt werden. Zu diesem Zweck wird als Entnahmevorrichtung ein Schälrohr 10 so in den Granulator eingebaut, daß die Schälrohröffnung 11 gerade in die Peripherie der Schüttguttrombe 9 eintaucht. Auf diese Weise kann das fertige Granulat mit dem überwiegend vorherrschenden größten Korndurchmesser kontinuierlich abgezogen werden. Die mittlere Korngröße des kontinuierlich entnommenen Granulatstromes läßt sich dabei über das zuvor eingestellte Mengenstromverhältnis von Pulver (Schneckendosierer 3) und Keimkorn (Schneckendosierer 4) steuern. Als Keimkorn kann entweder ein geeigneter Hilfsstoff eingesetzt werden (z. B. Milchzucker grob für pharmazeutische Präparate), oder ein in einem vorgeschalteten Granulationsprozeß erzeugtes Granulat der entsprechenden Korngröße (0,2 - 0,4 mm) verwendet werden.

Hohe Ansprüche an die Partikelgrößenverteilung machen bei dieser Betriebsweise eine Nachklassierung erforderlich.

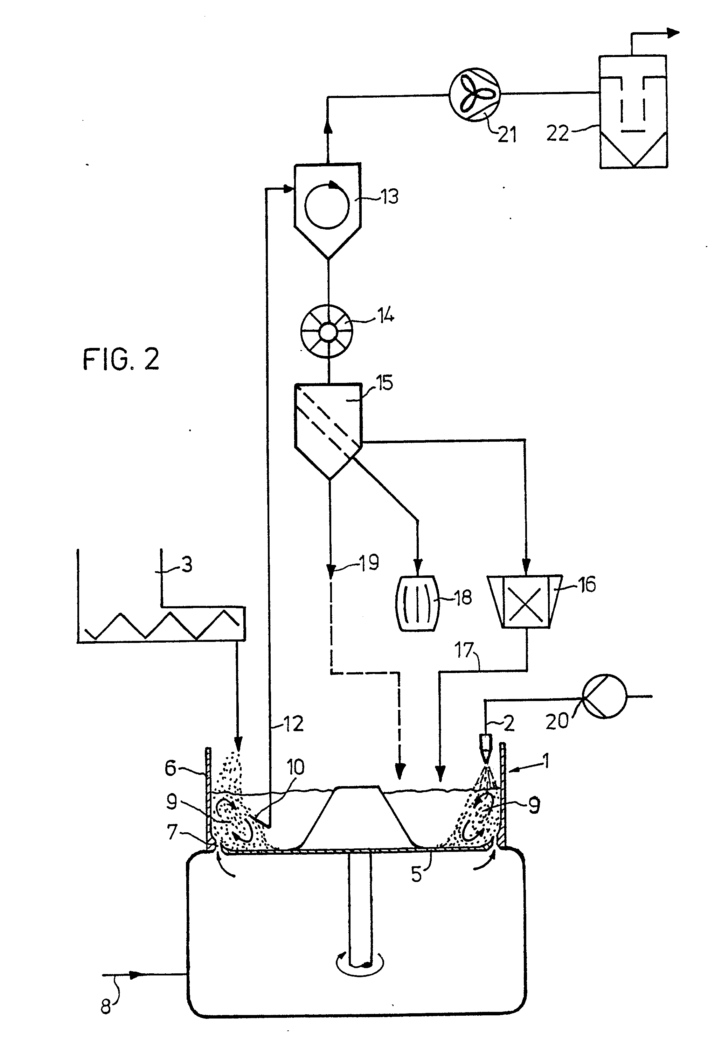

## b) Geschlossene Arbeitsweise (Fig. 2)

Wiederum aufbauend auf die im Stand der Technik beschriebene Zentraleinheit sind die folgenden Erweiterungen erforderlich:

-Das dem Granulator 1 durch das Schälrohr 10 entnommene vorklassierte Granulat wird durch die Leitung 12 pneumatisch zu einem Zyklonabscheider 13 gefördert, in dem noch vorhandene staubförmige Produktanteile abgetrennt werden. Der aus dem Zyklonabscheider 13 über eine Zellenradschleuse 14 austretende Produktstrom muß zur Trennung in drei Fraktionen (grob, mittel, fein) in einem extern angeordneten Klassierapparat 15, z.B. einem Fraktioniersieb, klassiert werden. Das Grobkorn wird in einem nachgeschalteten Passiersieb 16 zerkleinert und der zerkleinerte Grobgutstrom 17 als Keimgut in den Granulator 1 zurückgeführt. Die mittlere Fraktion ist das Fertiggranulat und wird in einen Vorratsbehälter 18 abgefüllt. Die Feinfraktion (Leitung 19) kann ebenfalls als Keimkorn in den Granulator 1 zurückgeführt werden.

Wie bei dem Ausführungsbeispiel nach Fig. 1 wird das zu granulierende Pulver über die Dosierschnecke 3 und die Granulierflüssigkeit über die Düse 2 (mit Dosierpumpe 20) zudosiert. Der Zyklonabscheider 13 steht über ein Gebläse 21 und ein Filter 22 zur Abscheidung von Feinstaub mit der Atmosphäre in Verbindung.

Bei der hier beschriebenen Arbeitsweise ist der Prozeß selbstregelnd. Eine Verringerung der mittleren Partikelgröße im Granulator führt zu einer Reduzierung des Grobanteiles im abgezogenen Granulat. Hierdurch wird der Keimkornmassenstrom (zerkleinertes Grobkorn) verringert, was ein Anwachsen der mittleren Partikelgröße im Granulator zur Folge hat.

Unabhängig von der Arbeitsweise des Granulators (kontinuierlich oder diskontinuierlich) benötigt das Verfahren eine produktabhängige mittlere Verweilzeit zum Einbinden des Pulvers. Wird der Massenstrom des Pulvers zu sehr erhöht (Verweilzeit verringert), so reichert sich im Granulator ungebundenes Pulver an, wodurch im kontinuierlichen Betrieb die Abrollbewegung der Granulate behindert wird, und das Verfahren versagt.

Bei konventionellen Rotorgranulatoren ist der Rotor 5 um die Vertikalachse drehbar, während das Gehäuse 6 feststehend ist. Einen größeren Spielraum (mehr Freiheitsgrade) hinsichtlich der Ausbildung der für die interne Klassierung kritischen Schüttguttrombe 9 kann man er reichen, wenn das Gehäuse 6 ebenfalls um die Längsachse drehbar ist und die Materialbewegung in der Schüttguttrombe 9 durch Einstellung der Differenzgeschwindigkeit zwischen Rotor 5 und Gehäuse 6 beeinflußt werden kann.

Das erfindungsgemäße Verfahren besitzt sehr breite Anwendungsmöglichkeiten.

In erster Linie kann es für die Herstellung von sphärischen Granulaten, human-pharmazeutischer oder verterinärpharmazeutischer Wirkstoffe angewandt werden.

Auch Pflanzenschutzmittel können unter Verwendung des erfindungsgemäßen Verfahrens formuliert werden. Weitere Anwendungsmöglichkeiten liegen in der Formulierung von Adsorbientien, Katalysatoren, Farbstoffen u.s.w. In allen Fällen der Anwendung des erfindungsgemäßen Verfahrens erhält man sphärische Granulate, die bei ihrer Verwendung den herkömmlichen Granulaten mit unbestimmter Oberfläche überlegen sind.

So können z. B. erfindungsgemäß hergestellte sphärische Granulate pharmazeutischer Wirkstoffe durch Lackierung sehr gezielt in Retardformulierungen übergeführt werden.

Beispiele

1. Zuckerpellets

In den Rotorgranulator werden gleichzeitig und kontinuierlich die Massenströme von Puderzucker, Wasser als Granulierflüssigkeit und Kristallzucker als Keimkorn in einem Mengenverhältnis von 1:5 bis 1:60 Keimkorn zu Pulver zudosiert. Die Granulierflüssigkeit wird über eine Zweistoffdüse im prozentualen Anteil zwischen 2 und 20 % bezogen auf Feststoff zugegeben. Nach Erreichen eines bestimmten Füllstands im Granulator wird über eine Entnahmesonde mit Hilfe der pneumatischen Förderung die gewünschte Kornfraktion klassierend aus der Kaskadenbewegung der Pellets aus dem Granulator entnommen. Die noch feuchten Granulate werden dann kontinuierlich oder batchweise in der Wirbelschicht getrocknet.

2. Hochdosierte human-pharmazeutische Zubereitungen

Die zu granulierenden, pulverförmigen Wirkstoffe z.B. Antirheumatika (Ketoprofen, Chlorquindiphosphat) oder Herztherapeutika vom Typ der Calciumantagonisten (Dihydropyridine) oder $\beta$-Blocker ($\alpha$-Methyldopa) werden im Gemisch mit einem ebenfalls pulverförmigen Bindemittel z. B. Polyvinylpyrrolidon oder kaltquellbare Maisstärke usw. im Verhältnis 1:300 bis 1:10 kontinuierlich in den Rotorgranulator dosiert und gleichzeitig Wasser als Granulierflüssigkeit über eine Ultraschalldüse im Verhältnis 2 bis 20 % bezogen auf Pulver zugegeben. Im stationären Betriebszustand laufen die Granulate mit gewünschtem Korndurchmesser vorklassiert über den Rand des Granulators ab und werden dann einer Nachklassiereinrichtung (z. B. Sieb oder Sichter) zugeführt. Die mittlere Fraktion (Gutkorn) wird online getrocknet. Das Überkorn wird einer geeigneten Zerkleinerungsvorrichtung (z. B. Backenbrecher, Rotationssieb, Passiersiebe usw.) zugeführt und gemeinsam mit dem Unterkorn getrocknet oder nicht getrocknet, abhängig vom Granulierverhalten des Produktes, als Keimkorn in den Rotorgranulator zurückgeführt. Zur Retardierung der Wirkstoffabgabe werden die Pellets z. B. in der Wirbelschicht oder in rotierenden Trommeln mit einer Diffusionsschicht in bekannter Weise überzogen. Die in Kapseln abgefüllten, so lackierten Pellets stellen Retardpräparate mit sehr definierter Wirkstofffreisetzung dar.

3. Hochdosierte veterinär-pharmazeutische Zubereitungen

Das zu granulierende Pulver eines antibiotischen Wirkstoffes aus der Klasse der Chinolone oder eines Wurmmittels, z.B. Praziquantel, wird gemeinsam mit dem pulverförmigen Bindemittel, z.B. Polyvinylpyrrolidon oder kaltquellbare Maisstärke oder andere, im Verhältnis 1:300 bis 1:10 kontinuierlich in den Rotorgranulator dosiert und gleichzeitig Wasser als Granulierflüssigkeit über eine Zweistoffdüse im Verhältnis 2 bis 20 % bezogen auf Pulver zugegeben. Im stationären Betriebszustand laufen die Granulate mit gewünschtem Korndurchmesser vorklassiert über den Rand des Granulators ab und werden dann einer Nachklassiereinheit (z. B. Sieb oder Sichter) zugeführt. Die mittlere Fraktion (Grobkorn) wird online getrocknet. Das Überkorn wird einer geeigneten Zerkleinerungsvorrichtung (z. B. Backenbrecher, Rotationssieb, Passiersieb usw.) zugeführt und gemeinsam mit dem Unterkorn getrocknet oder nicht getrocknet, abhängig vom Granulierverhalten des Produktes, als Keimkorn in den Rotorgranulator zurückgeführt. Weil die im Beispiel angegebenen Wirkstoffklassen einen sehr schlechten Geschmack besitzen, werden die Zubereitungen von den Tieren nur dann akzeptiert, wenn die Pellets mit einer geeigneten Lackschicht überzogen sind. Dies geschieht in bekannter weise z. B. in der Wirbelschicht. Die lackierten Pellets werden dem Futter der Tiere beigemischt.

## 4. Zeolithe

Sphärische Granulate mit der geforderten hohen Oberflächengüte und dem engen Kornspektrum werden erhalten, wenn pulverförmiges Aluminiumsilicat mit Wasserglas als Binder (z. B. 10 % bzw. auf den Pulveranteil) wie in obigen Beispielen beschrieben granuliert wird.

Ton-gebundene sphärische Zeolithe werden erhalten, wenn dem Aluminiumsilicat z. B. 10 % Tonmehl zugesetzt werden und anschließend mit Wasser wie oben beschrieben granuliert wird.

## 5. Pflanzenschutzmittel (z. B. Herbizid)

In einem externen Mischer werden die feinen Pulver folgender Komponenten in den Anteilen gemischt:
10 % Metribuzin
2 % Dibutylnaphthalinsulfonat
4 % Liquinsulfonat
3 % hochdisperse Kieselsäure
1 % Polyvinylpyrrolidon
40 % Kaolin
und anschließend dem Rotorgranulator kontinuierlich zudosiert. Gleichzeitig wird über eine Düse Wasser als Granulierflüssigkeit zudosiert, so daß das Feuchtgranulat eine Restfeuchte zwischen 5 - 20 % besitzt. Nach Erreichen eines bestimmten Füllstandes im Granulator wird über eine Entnahmesonde mit Hilfe der pneumatischen Förderung die gewünschte Kornfraktion klassierend aus der Kaskadenbewegung der Pellets aus dem Granulator entnommen. Nach kontinuierlicher Trocknung in einem Fließbetttrockner werden die Pellets im gleichen Apparat mit einer Polyvinylacetat-latex-suspension, Feststoffgehalt ca. 30 %ig, lackiert (30 - 60 % Susp. bezogen auf Pellets). So wird ein controlled release-Herbizid erhalten.

## 6. Träger für Katalysatoren

Siliciumdioxid, Aluminiumdioxid oder Tonmehl werden mit Wasser wie oben beschrieben im Rotor granuliert getrocknet und geglüht. Die erhaltenen sphärischen Träger können dann mit dem eigentlichen Katalysator beladen werden.

## Ansprüche

1) Verfahren zur kontinuierlichen Herstellung von sphärischem Granulat, bei dem in einem Rotorgranulator das in Form von Pulver und Keimkorn vorliegende Ausgangsmaterial durch Besprühen mit einer Flüssigkeit granuliert wird, dadurch gekennzeichnet, daß neben dem Pulver und der Granulierflüssigkeit auch das Keimkorn kontinuierlich zudosiert wird und das Granulat dem Granulator kontinuierlich entnommen wird.

2) Verfahren nach Anspruch 1, dadurch gekennzeichnet, daß mittels einer Entnahmevorrichtung das Granulat an der Peripherie einer am Innenumfang des Granulators umlaufenden Schüttguttrombe abgezogen wird, in der die Granulatpartikel eine zirkulierende Bewegung ausführen.

3) Verfahren nach Anspruch 1 oder 2, dadurch gekennzeichnet, daß eine Klassierung außerhalb des Granulators kontinuierlich erfolgt.

4) Verfahren nach Anspruch 3, dadurch gekennzeichnet, daß das Keimkorn durch externe Zerkleinerung einer Grobfrakton gewonnen und in den Granulator kontinuierlich rezirkuliert wird.

5) Verfahren nach Anspruch 1-2, dadurch gekennzeichnet, daß durch die Keimkorndosierrate bzw. durch die Partikelanzahl des Keimkornes die Korngröße des fertigen Granulates gesteuert werden kann.

6) Verfahren nach Anspruch 4, dadurch gekennzeichnet, daß durch Festlegung der Trennkorngröße bei der externen Fraktionierung das entstehende Kornspektrum des Granulats eingestellt wird und sich die hierfür erforderliche Keimkorndosierrate selbsttätig ergibt.

7) Verfahren nach Anspruch 4-6, dadurch gekennzeichnet, daß durch externe Zerkleinerung der Grobfraktion und komplette Rückführung die Ausbeute an Granulat der vorgegebenen Kornverteilung praktisch 100 % wird.

8) Verfahren nach Anspruch 1-7, dadurch gekennzeichnet, daß auch das Gehäuse des Granulators drehbar ausgeführt ist und Gehäuse und Rotor mit unterschiedlichen Drehzahlen betrieben werden.

FIG. 1

Le A 24 388

FIG. 2